(19) Europäisches Patentamt
European Patent Office
Office européen des brevets

(11) Veröffentlichungsnummer: **0 134 477**
**A1**

(12) # EUROPÄISCHE PATENTANMELDUNG

(21) Anmeldenummer: 84107831.4

(22) Anmeldetag: 05.07.84

(51) Int. Cl.⁴: **C 07 D 251/16**
C 07 D 239/42, A 01 N 47/36

(30) Priorität: 09.07.83 DE 3324800

(43) Veröffentlichungstag der Anmeldung: 20.03.85 Patentblatt 85/12

(84) *Benannte Vertragsstaaten:* AT BE CH DE FR GB IT LI NL SE

(71) Anmelder: HOECHST AKTIENGESELLSCHAFT
Postfach 80 03 20
D-6230 Frankfurt am Main 80(DE)

(72) Erfinder: Willms, Lothar, Dr.
Grüner Weg 4
D-5463 Unkel(DE)

(72) Erfinder: Humburg, Gerhard, Dr.
Königsberger Weg 24
D-6238 Hofheim am Taunus(DE)

(72) Erfinder: Bauer, Klaus, Dr.
Kolpingstrasse 7
D-6254 Rodgau(DE)

(72) Erfinder: Bieringer, Hermann, Dr.
Eichenweg 26
D-6239 Eppstein/Taunus(DE)

(72) Erfinder: Bürstell, Helmut, Dr.
Am Hohlacker 65
D-6000 Frankfurt am Main 50(DE)

(54) **Neue heterocyclisch substituierte Sulfonylharnstoffe.**

(57) Verbindungen der Formel (I)

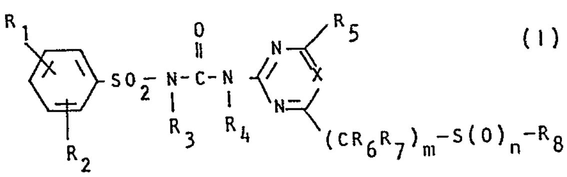

(I)

worin

$R_1$, $R_2$ unabhängig voneinander Wasserstoff, (subst.) Alkyl, (subst.) Alkoxy oder (subst.) Alkylthio, Halogen, $NO_2$, Alkoxycarbonyl, $(C_1-C_4)S(O)_n-$, $(R_9)(Alkyl)NSO_2-$, Alkenyloxy, Alkinyloxy, $-OSO_2$ Alkyl, $-OSO_2CF_3$, $-CONHR_3$, $-CON(R_3)_2$,

$R_5$ Wasserstoff, Halogen, Mono- oder Dialkylamino, (subst.) Alkyl, Alkoxy, Alkylthio, Allyloxy oder Propargyloxy,

$R_6$, $R_7$ unabhängig voneinander Wasserstoff, (subst.) Alkyl oder Halogen,

$R_8$ Alkyl, Alkenyl oder Benzyl,

$R_9$ (subst.) Alkyl oder Alkoxy,

m die Zahl 1 bis 3,

n die Zahl 0 bis 2,

X CH oder N bedeuten, sowie deren Salze besitzen vorteilhafte herbizide Eigenschaften gegen mono- und dikotyle Schadpflanzen. Sie eignen sich zum selektiven Einsatz in Großkulturen. Außerdem besitzen sie pflanzenwachstumsregulierende Wirkung.

EP 0 134 477 A1

Croydon Printing Company Ltd.

# Neue heterocyclisch substituierte Sulfonylharnstoffe

Es ist bereits bekannt, daß heterocyclisch substituierte Phenylsulfonylharnstoffe herbizide oder pflanzenwuchsregulierende Eigenschaften aufweisen, s. z.B. NL-PS 121 788, DE-OS 27 15 786, EP-PS 1 485.

Diese weisen jedoch Nachteile bei der Anwendung auf wie beispielsweise eine hohe Persistenz oder unzureichende Selektivität.

Es wurde nun gefunden, daß heterocyclisch substituierte Phenylsulfonylharnstoffe, die als heterocyclische Komponente einen mit einer Alkylthio-, Alkylsulfinyl- oder Alkylsulfonyl-alkyl-funktion substituierten Pyrimidin- bzw. Triazinring enthalten, als Herbizide und Pflanzenwachstumsregulatoren besonders geeignet sind.

Gegenstand der vorliegenden Erfindung sind daher Verbindungen der Formel (I)

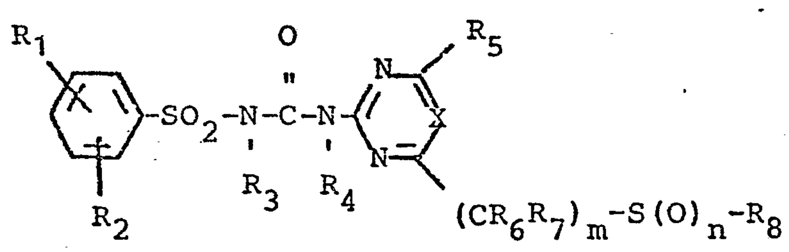

(I)

worin

$R_1$, $R_2$ unabhängig voneinander Wasserstoff, $(C_1-C_4)$-Alkyl, $(C_1-C_4)$-Alkoxy oder $(C_1-C_4)$-Alkylthio, die gegebenenfalls ein- oder mehrfach durch Halogen substituiert sind, Halogen, $NO_2$, $(C_1-C_4)$-Alkoxycarbonyl, $(C_1-C_4)S(O)_n-$, $(R_9)$ $(C_1-C_4$-Alkyl)N$SO_2$-

$(C_1-C_4)$-Alkenyloxy, $(C_1-C_4)$-Alkinyloxy, $-OSO_2-(C_1-C_4)$-Alkyl, $-OSO_2CF_3$, $-CONHR_3$, $-CON(R_3)_2$,

$R_3$, $R_4$ unabhängig voneinander Wasserstoff oder $(C_1-C_4)$-Alkyl,

$R_5$ Wasserstoff, Halogen, $(C_1-C_4)$-Alkylamino, Di-$(C_1-C_4)$-alkylamino, $(C_1-C_4)$-Alkyl, das gegebenenfalls ein oder mehrfach durch Halogen, $(C_1-C_3)$-Alkoxy oder durch $(C_1-C_3)$-Alkylthio substituiert ist, $(C_1-C_4)$-Alkoxy oder $(C_1-C_4)$-Alkylthio, Allyloxy oder Propargyloxy,

$R_6$, $R_7$ unabhängig voneinander Wasserstoff, $(C_1-C_4)$-Alkyl, $(C_1-C_4)$-Halogenalkyl, Halogen oder $(C_1-C_2)$-Alkoxy-$(C_1-C_2)$-alkyl,

$R_8$ $(C_1-C_4)$-Alkyl, $(C_2-C_4)$-Alkenyl oder Benzyl,

$R_9$ $(C_1-C_4)$-Alkyl, das gegebenenfalls durch CN oder $(C_1-C_4)$-Alkoxycarbonyl substituiert ist, oder $(C_1-C_4)$-Alkoxy,

m die Zahl 1 bis 3,

n die Zahl 0 bis 2,

X eine Methingruppe oder Stickstoff

bedeuten,

sowie die physiologisch verträglichen Salze dieser Verbindungen.

"Halogen" bedeutet bevorzugt Fluor, Chlor oder Brom.

Von den erfindungsgemäßen Verbindungen der allgemeinen Formel (I) sind diejenigen bevorzugt, bei denen $R_1$, $R_3$, $R_4$ Wasserstoff, $R_2$ die obengenannten Reste außer $(C_1-C_4)$-Alkyl, $(C_1-C_4)$-Alkoxy oder $(C_1-C_4)$-Alkylthio, $R_5$ $(C_1-C_4)$-Alkyl, das gegebenenfalls einfach oder mehrfach durch Halogen, $(C_1-C_3)$-Alkoxy, $(C_1-C_3)$-Alkylthio substituiert ist, $(C_1-C_4)$-Alkoxy, Allyloxy oder Propargyloxy, $R_6$, $R_7$ Wasserstoff oder $(C_1-C_2)$-Alkyl,

$R_8$ $(C_1-C_3)$-Alkyl und m die Zahl 1 oder 2 bedeuten und sich der $R_2$-Rest in ortho-Stellung des Phenylringes befindet.
Bevorzugt sind außerdem Verbindungen der Formel I, worin n=1 bedeutet.
Die neuen Verbindungen der allgemeinen Formel I lassen sich aus an sich bekannten oder nach bekannten Verfahren hergestellten Ausgangsmaterialien synthetisieren. Die Herstellungsverfahren sind dadurch gekennzeichnet, daß man

Verbindungen der Formel (II)

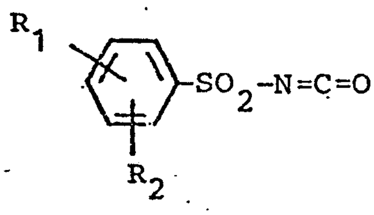

(II)

mit Verbindungen der Formel (III)

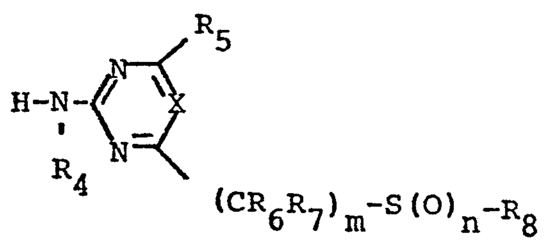

(III)

umsetzt, die erhaltenen Verbindungen der Formel I mit $R_3$ = H gegebenenfalls in $R_3$-Position alkyliert und gegebenenfalls im Falle n = 0 die erhaltenen Verbindungen der Formel I durch Umsetzung mit Oxidationsmitteln in die entsprechenden Sulfoxide der Formel I (n = 1) oder Sulfone der Formel I (n = 2) überführt.

Die Umsetzung der Verbindungen II und III erfolgt vorzugsweise in inerten aprotischen Lösungsmitteln wie z.B. Acetonitril, Dichlormethan, Toluol, Tetrahydrofuran oder Dioxan bei Temperaturen zwischen 0°C und der Siedetemperatur des Lösungsmittels.

Bei der Oxidation der Verbindungen I (n = 0) werden vorzugsweise Oxidationsmittel wie Kaliumpermangant, Wasserstoffperoxid oder m-Chlorperbenzoesäure in inerten Lösungsmitteln, wie z.B. Wasser oder Essigsäure (für $KMnO_4$ und $H_2O_2$) und z.B. Dichlormethan, oder Chloroform (für m-Chlorperbenzoesäure) - gegebenenfalls unter Zusatz von Katalysatoren wie z.B. Wolframsäure - bei Temperaturen zwischen -30°C und der Siedetemperatur des Lösungsmittels eingesetzt.

Zur nachträglichen Alkylierung der Verbindungen der Formel I mit $R_3$ = H arbeitet man vorzugsweise in inerten aprotischen Lösungsmitteln wie z.B. Dioxan oder Dimethylformamid unter Zusatz einer Base (wie z.B. Natriumhydrid oder Kaliumcarbonat) bei Temperaturen von 0°C bis zur Siedetemperatur des Lösungsmittels. Es werden übliche Alkylierungsmittel wie Dimethylsulfat, Methyljodid oder Ethylbromid eingesetzt.

Verbindungen der Formel I, in denen $R_3$ Wasserstoff bedeutet, können Salze bilden, bei denen der Wasserstoff durch ein für die Landwirtschaft geeignetes Kation ersetzt ist. Diese Salze sind im allgemeinen Metall-, insbesondere Alkali-, Erdalkali-, gebenenfalls substituierte, insbesondere alkylierte oder hydroxyalkylierte Ammonium- oder organische Aminsalze. Sie werden vorzugsweise in inerten Lösungsmitteln wie z.B. Wasser, Methanol oder Aceton bei Temperaturen von 20-100$^{O}$ C hergestellt. Geeignete Basen zur Herstellung der erfindungsgemäßen Salze sind beispielsweise Alkalicarbonate, wie Kaliumcarbonat, Ammoniak oder Ethanolamin.

Die Sulfonylisocyanate der Formel II sind zum großen Teil bekannt oder lassen sich nach im Prinzip bekannten Verfahren in einfacher Weise herstellen (vgl. EP-PS 15 683, EP-PS 21 641, US-PS 3 371 114).

Die Aminoheterocyclen der Formel III (X = N, n = O) sind bekannt oder lassen sich nach im Prinzip bekannten Verfahren herstellen, z.B. durch Cyclisierung entsprechender Guanidino-O-alkylisoharnstoffe mit Alkylmercaptocarbonsäureestern. (vgl. z.B. Chem. Pharm. Bull. 16, 474 (1968); J. pharm. Soc. Japan 95, 499 (1975)).

Die Aminopyrimidine der Formel (III) (X = CH, n = O) lassen sich nach prinzipiell bekannten Methoden durch Kondensation von Guanidin (- derivaten) mit entsprechend substituierten 1,3-Diketoverbindungen (vgl. z.B. "The Chemistry of Heterocyclic Compounds", Vol. XVI (1962) und Supplement I (1970) herstellen.

Die Sulfoxide und Sulfone der Formel (III) (n = 1, 2) lassen sich nach bekannten Methoden aus den voranstehend genannten Thioethern der Formel (III) (n = O) durch Oxidation, wie für die Verbindungen der Formel I mit n = O beschrieben, herstellen.

Die erfindungsgemäßen heterozyklischen Sulfonyharnstoffderivate weisen eine ausgezeichnete herbizide Wirkung und in wichtigen Großkulturen eine sehr gute Selektivität auf. Sie eignen sich daher zur selektiven Bekämpfung zweikeimblättriger und grasartiger annueller und perennierender Unkräuter, insbesondere in landwirtschaftlich bedeutenden Kulturen wie z.B. Weizen, Gerste, Roggen, Reis, Mais, Zuckerrübe und Soja. Dabei ist es gleichgültig, ob die Substanzen in Vorsaat-, Vorauflauf- oder Nachauflaufspritzung ausgebracht werden.

Werden die erfindungsgemäßen Verbindungen vor dem Keimen der Unkrautpflanzen im Vorsaat- oder Vorauflaufverfahren auf die Erdoberfläche appliziert, so wird das Auflaufen der Keimlinge nicht verhindert. Die Unkräuter wachsen bis zum Keimblattstadium heran, stellen jedoch dann ihr Wachstum ein und sterben schließlich nach 3 - 5 Wochen vollkommen ab. Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltstoffen und zur Ernteerleichterung wie z.B. durch Auslösen von Desikkation und Wuchsstauchung eingesetzt werden. Des weiteren eignen sich auch zur generellen Steuerung und Hemmung von unerwünschten vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann. Besonders hervorzuheben ist die wachstumsregulatorische Wirksamkeit der Verbindungen als Wuchshemmer in Getreide, Mais, Soja, Baumwolle, Rasen sowie ihre Fähigkeiten, den Gehalt an erwünschten Inhaltsstoffen wie Kohlehydraten und Protein bei Nutzpflanzen zu erhöhen. Schließlich zeigen die Verbindungen eine sehr gute Verbesserung der Fruchtabszission insbesondere bei Zitrusfrüchten oder Reduktion der Haltekraft.

Gegenstand der Erfindung sind daher auch herbizide bzw. wachstumsregulierende Mittel, die gekennzeichnet sind durch einen Gehalt an einer Verbindung der Formel I in Kombination mit üblichen Formulierungshilfsmitteln und Inertstoffen sowie deren Verwendung im landwirtschaftlichen Bereich.

Die erfindungsgemäßen Mittel enthalten die Wirkstoffe der Formel I im allgemeinen zu 2 - 95 Gew.-%. Sie können als Spritzpulver, emulgierbare Konzentrate, versprühbare Lösungen, Stäubemittel oder

Granulate in den üblichen Zubereitungen angewendet werden.

Die Spritzpulver sind in Wasser gleichmäßig dispergierbare Präparate, die neben dem Wirkstoff außer einem Verdünnungsmittel oder Inertstoff noch Netzmittel z.B. polyoxethylierte Alkylphenole, polyoxethylierte Fettalkohole, Alkyl- oder Alkylphenylsulfonate und Dispergiermittel, z.B. ligninsulfonsaures Natrium, 2,2'-dinaphthylmethan-6,6'-disulfonsaures Natrium, dibutylnaphthalinsulfonsaures Natrium oder auch oleylmethyltaurinsaures Natrium enthalten.

Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwandt werden:

Alkylarylsulfonsaure Kalziumsalze wie Ca-dodecylbenzosulfonat oder nichtionische Emulgatoren wie fettsäurepolyglykolester, Alkylarylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyether, Sorbitanfettsäureester, Polyoxethylensorbitfett-säureester oder Polyoxethylen-sorbitester.

Stäubemittel erhält man durch Vermahlen des Wirkstoffes mit fein verteilten, festen Stoffen, z.B. Talkum, natürlichen Tonen, wie Kaolin, Bentonit, Pyrophillit oder Diatomeenerde.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln z.B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranalien üblichen Weise - gewünschtenfalls in Mischung mit Düngemitteln - hergestellt werden.

Bei herbiziden Mitteln können die Konzentrationen der Wirkstoffe in den handelsüblichen Formulierungen verschieden sein.

In Spritzpulvern variiert die Wirkstoffkonzentration z.B. zwischen etwa 10 % und 80 %, der Rest besteht aus den oben angegebenen Formulierungszusätzen. Bei emulgierbaren Konzentraten kann die Wirkstoffkonzentration gleichfalls etwa 10 % bis 80 % betragen. Staubförmige Formulierungen enthalten etwa 2 - 20 %. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Zur Anwendung als Herbizide werden die handelsüblichen Konzentrate gegebenenfalls in üblicher Weise verdünnt, z.B. bei Spritzpulvern und emulgierbaren Konzentraten mittels Wasser. Staubförmige und granulierte Zubereitungen sowie versprühbare Lösungen werden vor der Anwendung nicht mehr mit weiteren inerten Stoffen verdünnt. Mit den äußeren Bedingungen wie Temperatur, Feuchtigkeit, u.a. variiert die erforderliche Aufwandmenge. Sie beträgt im allgemeinen zwischen 0.01 und 10 kg/ha, vorzugsweise etwa 0.1 bis 5.0 kg/ha Wirkstoff.

Für einige Anwendungsgebiete kann es zweckmäßig sein, die neuen Herbizide mit einem oder mehreren Herbiziden gemeinsam anzuwenden, z.B. als Tankmischung oder in Form einer Fertigformulierung, um weitere vorteilhafte Wirkungen zu erzielen.

Die erfindungsgemäßen Wirkstoffe können mit anderen Herbiziden, Insektiziden und Fungiziden kombiniert werden.

Zur Anwendung als Wachstumsregulatoren eignen sich Konzentrationen zwischen 0.01 und 1.25 kg/ha. Bevorzugt verwendet man wäßrige Dispersionen von Spritzpulvern oder Verdünnungen von emulgierbaren Konzentraten. Die Anwendung erfolgt im Nachauflauf. Bevorzugte Kulturen sind Mais und Tabak.

HERSTELLUNGSBEISPIELE

Beispiel 1

2-Amino-4-methoxy-6-methylthiomethyl-1,3,5-triazin

15,2 (0,1 Mol) Guanyl-O-methylisoharnstoff-Hydrochlorid - gelöst in 80 ml Methanol - werden bei 0°C in eine Lösung von 2,53 g (0,11 Mol) Natrium in 40 ml Methanol getropft. Nach 1 Stunde Rühren bei Raumtemperatur wird der Rückstand abfiltriert und das Filtrat bei 0°C mit 13,5 g (0,1 Mol) Methylthioessigsäureethylester - in 20 ml Methanol gelöst - versetzt. Nach 18 Stunden Rühren bei Raumtemperatur wird eingedampft, der Rückstand mit Wasser verrieben und getrocknet. Man erhält 9,5 g (52 % d.Th.) an 2-Amino-4-methoxy-6-methylthiomethyl-1,3,5-triazin vom Smp. 155 - 158°C.

Beispiel 2

2-Amino-4-methoxy-6-methylsulfonylmethyl-1,3,5-triazin

9,3 g (0,05 Mol) 2-Amino-4-methoxy-6-methylthio-methyl-1,3,5-triazin (vgl. Beispiel 1) werden in 100 ml Eisessig gelöst und bei 10 - 15°C mit 17,4 g (0,11 Mol) $KMnO_4$ - gelöst in 500 ml Wasser - innerhalb von 2 Stunden versetzt. Anschließend wird bis zur Entfärbung bei 0 - 5°C Schwefeldioxid eingeleitet, das ausgefallene Produkt abgesaugt und getrocknet. Man erhält 7 g (64,2 % d.Th.) an 2-Amino-4-methoxy-6-methylsulfonylmethyl-1,3,5-triazin vom Smp. 204 - 206°C.

Beispiel 3

N-(2-Chlorphenylsulfonyl)-N'-(4-methylthiomethyl-6-methoxy-1,3,5-triazin-2-yl)-harnstoff

Zu 5,58 g (0,03 Mol) 2-Amino-4-methylthiomethyl-6-methoxy-1,3,5-triazin in 100 ml Dichlormethan tropft man bei 0 bis 5°C eine Lösung von 6,5 g (0,03 Mol) 2-Chlorphenylsulfonylisocyanat in 20 ml Dichlormethan. Man rührt 18 h bei Raumtemperatur nach, setzt n-Hexan hinzu und saugt das angefallene Reaktionsprodukt ab. Man erhält 9,62 g (79,5 % d.Th.) an N-(2-Chlorphenylsulfonyl)-N'-(4-methylthiomethyl-6-methoxy-1,3,5-triazin-2-yl)-harnstoff vom Schmelzpunkt 124 bis 127°C.

Beispiel 4

N-(2-Chlorphenylsulfonyl)-N'-(4-methylsulfinylethyl-6-methoxy-1,3,5-triazin-2-yl)-harnstoff

12,5 g (0,03 Mol) N-(2-Chlorphenylsulfonyl)-N'-(4-methylmercaptoethyl-6-methoxy-1,3,5-triazin-2-yl)-harnstoff (vergleiche Beispiel 72) werden in 150 ml Dichlormethan gelöst und bei - 20°C mit einer Lösung von 6,1 g (0,03 Mol) m-Chlorperbenzoesäure (ca. 85 %ig) in 50 ml $CH_2Cl_2$ versetzt. Man rührt 18 h bei Raumtemperatur nach, setzt n-Hexan hinzu und saugt das Reaktionsprodukt ab. Man erhält 9,1 g (70 % d.Th.) an N(2-Chlorphenyl-sulfonyl)-N'-(4-methylsulfinylethyl-6-methoxy-1,3,5-triazin-2-yl)-harnstoff vom Schmelzpunkt 122 bis 124°C.

0134477

Auf analoge Weise werden die folgenden Verbindungen hergestellt (Tabelle 1 und 2).

Tabelle 1:

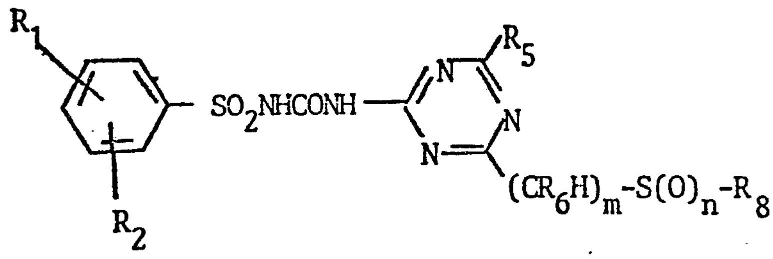

| Verb.-Nr. | R2 | R1 | R5 | R6 | m | n | R8 | Smp. |
|---|---|---|---|---|---|---|---|---|
| 5 | 2-$NO_2$ | H | $OCH_3$ | H | 1 | 0 | $CH_3$ | 118 - 120 |
| 6 | 2-$OCF_2H$ | H | $OCH_3$ | H | 1 | 0 | $CH_3$ | 112 - 114 |
| 7 | 2-$CH_3$ | H | $OCH_3$ | H | 1 | 0 | $CH_3$ | 138 |
| 8 | 2-$CF_3$ | H | $OCH_3$ | H | 1 | 0 | $CH_3$ | |
| 9 | 2-$CCl_3$ | H | $OCH_3$ | H | 1 | 0 | $CH_3$ | |
| 10 | 2-$COOCH_3$ | H | $OCH_3$ | H | 1 | 0 | $CH_3$ | 151 |
| 11. | 2-$SO_2N(CH_3)_2$ | H | $OCH_3$ | H | 1 | 0 | $CH_3$ | 60 - 64 |
| 12 | 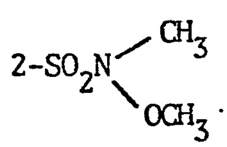 | H | $OCH_3$ | H | 1 | 0 | $CH_3$ | |
| 13 | 2-$SO_2CH_3$ | H | $OCH_3$ | H | 1 | 0 | $CH_3$ | |
| 14 | 2-$CHCl_2$ | H | $OCH_3$ | H | 1 | 0 | $CH_3$ | |
| 15 | 2-$OSO_2CH_3$ | H | $OCH_3$ | H | 1 | 0 | $CH_3$ | |
| 16 | 2-Cl | H | $CH_3$ | H | 1 | 0 | $CH_3$ | |

Tabelle 1: (Fortsetzung)

| Verb.-Nr. | $R_2$ | $R_1$ | $R_5$ | $R_6$ | m | n | $R_8$ | Smp. |
|---|---|---|---|---|---|---|---|---|
| 17 | 2-$COOCH_3$ | H | $CH_3$ | H | 1 | 0 | $CH_3$ | |
| 18 | 2-$OCH_2CH{=}CH_2$ | H | $CH_3$ | H | 1 | 0 | $CH_3$ | |
| 19 | 2-$OCH_2$-$C{\equiv}CH$ | H | $CH_3$ | H | 1 | 0 | $CH_3$ | |
| 20 | 2-$SCH_3$ | H | $CH_3$ | H | 1 | 0 | $CH_3$ | |
| 21 | 2-$OCH_3$ | H | $CH_3$ | H | 1 | 0 | $CH_3$ | |
| 22 | 2-Cl | 6-Cl | $OCH_3$ | H | 1 | 0 | $CH_3$ | 132 - 134 |
| 23 | 2-Cl | 5-$CF_3$ | $OCH_3$ | H | 1 | 0 | $CH_3$ | |
| 24 | 2-$CF_3$ | 4-$CH_3$ | $OCH_3$ | H | 1 | 0 | $CH_3$ | |
| 25 | 2-Cl | 3-Cl | $OCH_3$ | H | 1 | 0 | $CH_3$ | |
| 26 | 2-Cl | H | $OCH_3$ | H | 1 | 0 | $C_2H_5$ | 125 - 127 |
| 27 | 2-$OCF_2H$ | H | $OCH_3$ | H | 1 | 0 | -$CH(CH_3)_2$ | |
| 28 | 2-$COOCH_3$ | H | $OCH_3$ | H | 1 | 0 | $C(CH_3)_3$ | |
| 29 | 2-Cl | H | $OCH_3$ | $CH_3$ | 1 | 0 | $CH_3$ | 157 - 158 |
| 30 | 2-$COOCH_3$ | H | $OCH_3$ | $CH_3$ | 1 | 0 | $CH_3$ | 133 - 135 |
| 31 | 2-Cl | H | $OC_2H_5$ | $CH_3$ | 1 | 0 | $CH_3$ | |
| 32 | 2-Cl | H | $OC_2H_5$ | $CH_3$ | 1 | 0 | $C_2H_5$ | 58 - 60 |
| 33 | 2-Cl | H | $OC_2H_5$ | H | 1 | 0 | $CH_3$ | 115 - 118 |
| 34 | 2-$COOCH_3$ | H | $OC_2H_5$ | H | 1 | 0 | $CH_3$ | |
| 35 | 2-$OCF_2H$ | H | $OC_2H_5$ | H | 1 | 0 | $CH_3$ | |
| 36 | 2-Cl | H | $OCH_3$ | H | 1 | 0 | $CH_3$ | |
| 37 | 2-Cl | H | $OCH_3$ | H | 1 | 0 | $CH_2$-$CH{=}CH_2$ | |
| 38 | 2-F | H | $OCH_3$ | H | 1 | 0 | $CH_3$ | |

Tabelle 1: (Fortsetzung)

| Verb.-Nr. | $R_2$ | $R_1$ | $R_5$ | $R_6$ | m | n | $R_8$ | Smp. |
|---|---|---|---|---|---|---|---|---|
| 39 | 2-Cl | H | $OCH_3$ | H | 1 | 2 | $CH_3$ | 116 - 118 |
| 40 | 2-Cl | H | $OCH_3$ | $CH_3$ | 1 | 2 | $CH_3$ | 172 - 174 |
| 41 | 2-$OCF_2H$ | H | $OCH_3$ | H | 1 | 2 | $CH_3$ | 132 - 135 |
| 42 | 2-$CF_3$ | H | $OCH_3$ | H | 1 | 2 | $CH_3$ | |
| 43 | 2-$SO_2CH_3$ | H | $OCH_3$ | H | 1 | 2 | $CH_3$ | |
| 44 | 2-Cl | H | $OC_2H_5$ | H | 1 | 2 | $CH_3$ | 140 - 143 |
| 45 | 2-Cl | H | $OCH_3$ | H | 1 | 2 | $C_2H_5$ | 165 - 167 |
| 46 | 2-$COOCH_3$ | H | $OCH_3$ | H | 1 | 2 | $CH_3$ | 152 - 157 |
| 47 | 2-Cl | H | $OC_2H_5$ | H | 1 | 2 | $C_2H_5$ | |
| 48 | 2-Cl | H | $OCH_3$ | H | 1 | 2 | $CH(CH_3)_2$ | |
| 49 | 2-Cl | 4-Cl | $OCH_3$ | H | 1 | 2 | $CH_3$ | |
| 50 | 2-$COOC_2H_5$ | H | $OCH_3$ | H | 1 | 2 | $CH_3$ | |
| 51 | 2-$COOCH_3$ | H | $OC_3H_7$ | H | 1 | 2 | $CH_3$ | |
| 52 | 2-$COOCH_3$ | H | $CH(CH_3)_2$ | H | 1 | 2 | $CH_3$ | |
| 53 | 2-$OCF_2H$ | H | $OC_2H_5$ | H | 1 | 2 | $C_2H_5$ | 73 - 74 |
| 54 | 2-$SC_2H_5$ | H | $CH_3$ | H | 1 | 2 | $CH_3$ | |
| 55 | 2-Cl | H | $OCH_3$ | H | 1 | 1 | $CH_3$ | 100 - 101(Z) |
| 56 | 2-$OCF_2H$ | H | $OCH_3$ | H | 1 | 1 | $CH_3$ | 134 - 136 |
| 57 | 2-$COOCH_3$ | H | $OCH_3$ | H | 1 | 1 | $CH_3$ | 118 - 122 |
| 58 | 2-$CF_3$ | H | $OCH_3$ | H | 1 | 1 | $CH_3$ | |
| 59 | 2-Cl | H | $OC_2H_5$ | H | 1 | 1 | $CH_3$ | |

Tabelle 1: (Fortsetzung)

| Verb.-Nr. | $R_2$ | $R_1$ | $R_5$ | $R_6$ | m | n | $R_8$ | Smp. |
|---|---|---|---|---|---|---|---|---|
| 61 | 2-$COOCH_3$ | 5-$CH_3$ | $OC_2H_5$ | H | 1 | 1 | $CH_3$ | |
| 62 | 2-$COOCH_3$ | H | $OCH_3$ | $CH_3$ | 1 | 1 | $CH_3$ | |
| 63 | 2-$SO_2CH_3$ | H | $OCH_3$ | $CH_3$ | 1 | 1 | $CH_3$ | |
| 64 | 2-$SO_2CH_3$ | H | $OCH_3$ | | 1 | 1 | $C_2H_5$ | |
| 65 | 2-$OSO_2CH_3$ | H | $OCH_3$ | | 1 | 1 | $CH_3$ | |
| 66 | 2-$OSO_2CH_3$ | H | $OCH_3$ | | 1 | 1 | $CH(CH_3)_2$ | |
| 67 | 2-$SO_2CH_3$ | H | $OCH_3$ | | 1 | 1 | $-CH_2-$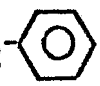 | |
| 68 | 2-Cl | 6-Cl | $OCH_3$ | | 1 | 1 | $CH_3$ | |
| 69 | 2-Cl | 4-Cl | $OCH_3$ | | 1 | 1 | $CH_3$ | |
| 70 | 2-F | H | $OCH_3$ | H | 1 | 1 | $CH_3$ | |
| 71 | 2-Br | H | $CH_3$ | H | 1 | 1 | $CH_3$ | |
| 72 | 2-Cl | H | $OCH_3$ | H | 2 | 0 | $CH_3$ | 128 - 131 |
| 73 | 2-Cl | H | $OCH_3$ | H | 2 | 2 | $CH_3$ | 143 - 147 |
| 74 | 2-Cl | H | $OCH_3$ | H | 2 | 0 | $C_2H_5$ | |
| 75 | 2-$COOCH_3$ | H | $OCH_3$ | H | 2 | 0 | $CH_3$ | |
| 76 | 2-$CF_3$ | 5-$CH_3$ | $OCH_3$ | H | 2 | 0 | $CH_3$ | |
| 77 | 2-$SO_2CH_3$ | H | $OCH_3$ | H | 2 | 0 | $CH_3$ | |
| 78 | 2-$SO_2N(CH_3)_2$ | H | $OCH_3$ | H | 2 | 0 | $CH_3$ | |
| 79 | 2-Cl | H | $OC_2H_5$ | H | 2 | 0 | $CH_3$ | |
| 80 | 2-Cl | H | $OCH_3$ | $CH_3$ | 2 | 0 | $CH_3$ | |
| 81 | 2-$COOCH_3$ | H | $OCH_3$ | $CH_3$ | 2 | 0 | $CH_3$ | |

Tabelle 1: (Fortsetzung)

| Verb.-Nr. | $R_2$ | $R_1$ | $R_5$ | $R_6$ | m | n | $R_8$ | Smp. |
|---|---|---|---|---|---|---|---|---|
| 82 | 2-Cl | H | $OCH_3$ | $CH_3$ | 2 | 1 | $CH_3$ | |
| 83 | 2-$COOCH_3$ | H | $OCH_3$ | $CH_3$ | 2 | 1 | $CH_3$ | |
| 84 | 2-$COOCH_3$ | H | $OCH_3$ | H | 2 | 1 | $CH_3$ | |
| 85 | 2-$OCF_2H$ | H | $OCH_3$ | H | 2 | 0 | $CH_3$ | |
| 86 | 2-$OCF_2H$ | H | $OCH_3$ | H | 2 | 1 | $CH_3$ | |
| 87 | 2-$OCF_2H$ | H | $OCH_3$ | H | 2 | 2 | $CH_3$ | |
| 88 | 2-$OCF_2H$ | H | $OCH_3$ | H | 3 | 0 | $CH_3$ | |

Tabelle 2:

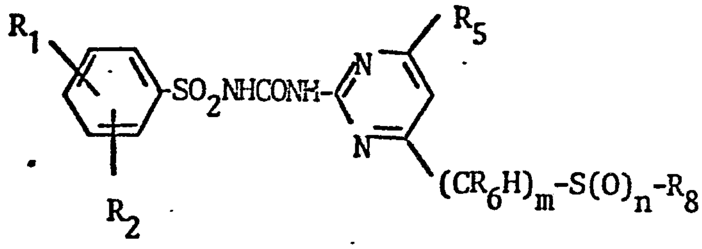

| Verb.-Nr. | $R_2$ | $R_1$ | $R_5$ | $R_6$ | m | n | $R_8$ | Smp. |
|---|---|---|---|---|---|---|---|---|
| 89 | 2-Cl | H | $CH_3$ | H | 1 | 0 | $CH_3$ | |
| 90 | 2-Cl | H | $OCH_3$ | H | 1 | 0 | $CH_3$ | 140 - 142 |
| 91 | 2-Cl | H | $OCH_3$ | H | 1 | 1 | $CH_3$ | 124 - 126(Z) |
| 92 | 2-Cl | H | $OCH_3$ | H | 1 | 2 | $CH_3$ | 158 - 190 |
| 93 | 2-Cl | H | $OC_2H_5$ | H | 1 | 0 | $CH_3$ | |
| 94 | 2-Cl | H | $OC_2H_5$ | H | 1 | 0 | $CH_3$ | |
| 95 | 2-Cl | H | $CH_3$ | $CH_3$ | 1 | 0 | $CH_3$ | |
| 96 | 2-Cl | H | $OCH_3$ | $C_2H_5$ | 1 | 0 | $CH_3$ | |
| 97 | 2-Cl | H | $OCH_3$ | H | 2 | 0 | $CH_3$ | |
| 98 | 2-$COOCH_3$ | H | $OCH_3$ | H | 1 | 0 | $CH_3$ | |
| 99 | 2-$COOCH_3$ | H | $OC_2H_5$ | H | 1 | 0 | $CH_3$ | |
| 100 | 2-$COOCH_3$ | H | $CH_3$ | H | 1 | 0 | $CH_3$ | |
| 101 | 2-$OCF_2H$ | H | $CH_3$ | H | 1 | 0 | $CH_3$ | |
| 102 | 2-$OCF_2H$ | H | $OCH_3$ | H | 1 | 0 | $CH_3$ | |
| 103 | 2-$SO_2CH_3$ | H | $OCH_3$ | H | 1 | 0 | $CH_3$ | |
| 104 | 2-F | H | $OCH_3$ | H | 1 | 0 | $CH_3$ | |

0134477

Tabelle 2: (Fortsetzung)

| Verb.-Nr. | $R_2$ | $R_1$ | $R_5$ | $R_6$ | m | n | $R_8$ | Smp. |
|---|---|---|---|---|---|---|---|---|
| 105 | 2-$CF_3$ | 5-$CH_3$ | $OCH_3$ | H | 1 | 0 | $CH_3$ | |
| 106 | 2-Cl | H | Cl | H | 1 | 0 | $CH_3$ | |
| 107 | 2-$COOCH_3$ | H | Cl | H | 1 | 0 | $CH_3$ | |
| 108 | 2-Cl | H | H | H | 1 | 0 | $CH_3$ | |
| 109 | 2-Cl | H | $NHCH_3$ | H | 1 | 0 | $CH_3$ | |
| 110 | 2-Cl | H | $N(CH_3)_2$ | H | 1 | 0 | $CH_3$ | |
| 111 | 2-Cl | H | $CH_2Cl$ | H | 1 | 0 | $CH_3$ | |
| 112 | 2-Cl | H | $CH_2OCH_3$ | H | 1 | 0 | $CH_3$ | |
| 113 | 2-Cl | H | $CH_2SCH_3$ | H | 1 | 0 | $CH_3$ | |
| 114 | 2-Cl | H | $SCH_3$ | H | 1 | 0 | $CH_3$ | |
| 115 | 2-Cl | H | $(-OCH_2CH{=}CH_2)$ | H | 1 | 0 | $CH_3$ | |
| 116 | 2-Cl | H | $(-O-CH_2C{\equiv}CH)$ | H | 1 | 0 | $CH_3$ | |
| 117 | 2-$COOCH_3$ | H | $OCH_3$ | H | 2 | 0 | $CH_3$ | |
| 118 | 2-$COOCH_3$ | H | $OCH_3$ | H | 2 | 2 | $CH_3$ | |
| 119 | 2-$COOCH_3$ | H | $OCH_3$ | H | 2 | 0 | $C_2H_5$ | |
| 120 | 2-$COOCH_3$ | H | $OCH_3$ | H | 2 | 1 | $C_2H_5$ | |
| 121 | 2-$COOCH_3$ | H | $OCH_3$ | $CH_3$ | 2 | 1 | $CH_3$ | |
| 122 | 2-$COOCH_3$ | 5-$CH_3$ | $OCH_3$ | $CH_3$ | 2 | 0 | $CH_3$ | |

I. Herbizide Wirkung

Die vorliegenden erfindungsgemäßen Verbindungen weisen eine ausgezeichnete herbizide Wirksamkeit gegen ein breites Spektrum wirtschaftlich wichtiger mono- und dikotyler Schadpflanzen auf. Auch schwer bekämpfbare perennierende Wurzelunkräuter werden durch die Wirkstoffe gut erfaßt. Dabei ist es gleichgültig, ob die Substanzen in Vorsaat-, Vorauflauf- oder Nachauflaufspritzung ausgebracht werden.

Werden die erfindungsgemäßen Verbindungen vor dem Keimen der Unkrautpflanzen im Vorsaat- oder Vorauflaufverfahren auf die Erdoberfläche appliziert, so wird das Auflaufen der Keimlinge nicht verhindert. Die Unkräuter wachsen bis zum Keimblattstadium heran, stellen jedoch dann ihr Wachstum ein und sterben schließlich nach 3 - 5 Wochen vollkommen ab. Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig durch den Einsatz der neuen erfindungsgemäßen Mittel beseitigt werden kann.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Gegenüber dem Stand der Technik besitzen die vorliegenden erfindungsgemäßen Substanzen deshalb eine wesentlich verbesserte Selektivität bei Kulturpflanzen.

Die vorliegenden Verbindungen eignen sich aus diesen Gründen sehr gut zur Bekämpfung von unerwünschtem Pflanzenwuchs in landwirtschaftlichen Nutzpflanzungen.

Die Schädigung der Unkrautpflanzen bzw. die Kulturpflanzenverträglichkeit wurde in einem Schlüssel von 0 - 5 bonitiert.

Dabei bedeutet

0 = ohne Wirkung (Schaden)
1 = 0 - 20 % Wirkung
2 = 20 - 40 % Wirkung
3 = 40 - 60 % Wirkung
4 = 60 - 80 % Wirkung
5 = 80 - 100 % Wirkung

1. Vorauflaufverfahren

Samen bzw. Rhizomstücke mono- und dikotyler Unkräuter wurden in Lehmerde in Plastiktöpfen (Ø 9 cm) ausgelegt und mit Erde abgedeckt. Die als benetzbare Pulver bzw. als Emulsionskonzentrate formulierten erfindungsgemäßen Verbindungen wurden in Form wässriger Suspensionen oder Emulsionen auf die Erdoberfläche appliziert. Die Wasseraufwandmenge pro Topf entsprach dabei umgerechnet 600 - 800 l/ha. Nach der Behandlung wurden die Versuchstöpfe im Gewächshaus aufgestellt und die Versuchspflanzen unter guten Wachstumsbedingungen (Temperatur: 23 ± 1°C; rel. Luftfeuchte 60 - 80 %) kultiviert. Nach ca. 3 Wochen wurde die Pflanzenschädigung optisch bonitiert. Als Vergleich dienten dabei unbehandelte Kontrollen. In der Tabelle 3 sind die Vorauflaufergebnisse zusammengefaßt.
Die dargestellten Versuchsergebnisse belegen die ausgezeichnete herbizide Vorauflaufwirkung der neuen, erfindungsgemäßen Verbindungen gegen Ungräser und Unkräuter.

2. Nachauflaufverfahren

Samen von mono- und dikotylen Unkräutern wurden in Töpfen ausgesät und im Gewächshaus unter guten Wachstumsbedingungen angezogen. 3 Wochen nach der Aussaat wurden die Versuchspflanzen im Dreiblattstadium behandelt. Die als Spritzpulver bzw. als Emulsionskonzentrate formulierten erfindungsgemäßen Präparate wurden in verschiedenen Dosierungen auf die grünen Pflanzenteile gesprüht und nach ca. 3 Wochen Standzeit im Gewächshaus unter optimalen Wachstumsbedingungen (Temperatur: 23 ± 1°C; rel. Luftfeuchte 60 - 80 %) die Wirkung der Präparate optisch im Vergleich zu unbehandelten Kontrollen bonitiert. Die erfindungsgemäßen Mittel weisen eine gute herbizide Wirksamkeit gegen ein breites Spektrum wirtschaftlich wichtiger annueller und perennierender Unkräuter und Ungräser auf (Tabelle 4).

Tabelle 3: Herbizide Wirkung der Verbindungen (I) im Vorauflauf

| Verbindungs-Nr. | Dosis kg a.i./ha | herbizide Wirkung STM | AMR | SIA | LOM | ECG | AS |
|---|---|---|---|---|---|---|---|
| 3 | 2,5 | 5 | 5 | 5 | 5 | 5 | 1 |
| 4 | 2,5 | 5 | 5 | 5 | 5 | 5 | 2 |
| 5 | 2,5 | 5 | 5 | 5 | 4 | 5 | 3 |
| 6 | 2,5 | 5 | 5 | 5 | 5 | 4 | 3 |
| 7 | 2,5 | 5 | 5 | 5 | 5 | 5 | 3 |
| 10 | 2,5 | 5 | 5 | 5 | 5 | 5 | 3 |
| 11 | 2,5 | 5 | 5 | 5 | 5 | 5 | 4 |
| 26 | 2,5 | 5 | 5 | 5 | 5 | 5 | 4 |
| 29 | 2,5 | 5 | 5 | 5 | 5 | 5 | 2 |
| 30 | 2,5 | 5 | 5 | 5 | 5 | 5 | 4 |
| 39 | 2,5 | 5 | 5 | 5 | 5 | 5 | 1 |
| 40 | 2,5 | 5 | 5 | 5 | 5 | 5 | 1 |
| 41 | 2,5 | 5 | 5 | 5 | 5 | 5 | 2 |
| 44 | 2,5 | 5 | 5 | 5 | 4 | 5 | 4 |
| 45 | 2,5 | 5 | 5 | 5 | 4 | 5 | 1 |
| 46 | 2,5 | 5 | 5 | 5 | 4 | 5 | 5 |
| 33 | 2,5 | 5 | 5 | 5 | 5 | 5 | 2 |
| 55 | 2,5 | 5 | 5 | 5 | 5 | 5 | 4 |
| 56 | 2,5 | 5 | 5 | 4 | 5 | 5 | 3 |
| 72 | 2,5 | 5 | 5 | 5 | 5 | 5 | 4 |
| 73 | 2,5 | 5 | 5 | 5 | 5 | 5 | 5 |
| 90 | 2,5 | 5 | 5 | 5 | 5 | 5 | 4 |
| 91 | 2,5 | 5 | 5 | 5 | 5 | 5 | 5 |
| 92 | 2,5 | 5 | 5 | 5 | 5 | 5 | 4 |
| 57 | 2,5 | 5 | 4 | 5 | 5 | 5 | 5 |

Tabelle 4: Herbizide Wirkung der Verbindungen (I) im Nachauflauf.

| Verbindungs-Nr. | Dosis kg a.i./ha | herbizide Wirkung SIA | AMR | STM | AS | ECG | LOM |
|---|---|---|---|---|---|---|---|
| 3 | 2,5 | 5 | 5 | 5 | 5 | 5 | 3 |
| 4 | 2,5 | 5 | 5 | 5 | 5 | 5 | 2 |
| 5 | 2,5 | 5 | 5 | 5 | 5 | 5 | 5 |
| 6 | 2,5 | 5 | 4 | 4 | 3 | 5 | 4 |
| 7 | 2,5 | 5 | 5 | 5 | 4 | 5 | 5 |
| 10 | 2,5 | 5 | 5 | 5 | 4 | 5 | 5 |
| 11 | 2,5 | 5 | 5 | 5 | 5 | 5 | 5 |
| 26 | 2,5 | 5 | 5 | 5 | 2 | 5 | 5 |
| 29 | 2,5 | 5 | 2 | 5 | 2 | 3 | 4 |
| 30 | 2,5 | 5 | 5 | 5 | 3 | 4 | 5 |
| 39 | 2,5 | 5 | 5 | 5 | 2 | 5 | 5 |
| 40 | 2,5 | 5 | 5 | 5 | 1 | 5 | 5 |
| 41 | 2,5 | 5 | 5 | 5 | 3 | 4 | 5 |
| 44 | 2,5 | 5 | 5 | 5 | 2 | 5 | 4 |
| 45 | 2,5 | 5 | 5 | 5 | 2 | 5 | 5 |
| 46 | 2,5 | 5 | 5 | 5 | 3 | 4 | 5 |
| 33 | 2,5 | 5 | 5 | 5 | 4 | 5 | 4 |
| 55 | 2,5 | 5 | 5 | 5 | 4 | 5 | 3 |
| 56 | 2,5 | 5 | 5 | 5 | 4 | 4 | 2 |
| 72 | 2,5 | 5 | 5 | 5 | 4 | 5 | 2 |
| 73 | 2,5 | 5 | 5 | 4 | 3 | 4 | 4 |
| 90 | 2,5 | 5 | 5 | 5 | 4 | 5 | 5 |
| 91 | 2,5 | 5 | 5 | 5 | 5 | 5 | 5 |
| 92 | 2,5 | 5 | 5 | 5 | 4 | 5 | 4 |
| 57 | 2,5 | 5 | 4 | 5 | 5 | 5 | 5 |

Patentansprüche:

1. Verbindungen der Formel I

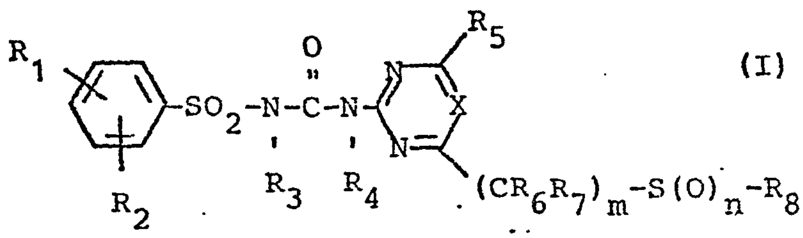

(I)

worin

$R_1$, $R_2$ unabhängig voneinander Wasserstoff, $(C_1-C_4)$-Alkyl, $(C_1-C_4)$-Alkoxy oder $(C_1-C_4)$-Alkylthio, die gegebenenfalls ein- oder mehrfach durch Halogen substituiert sind, Halogen, $NO_2$, $(C_1-C_4)$-Alkoxy-carbonyl, $(C_1-C_4)S(O)_n$-, $-SO_2N(R_9)$ $(C_1-C_4$-Alkyl), $(C_1-C_4)$-Alkenyloxy, $(C_1-C_4)$-Alkinyloxy, $-OSO_2-(C_1-C_4)$-Alkyl, $-OSO_2CF_3$, $-CONHR_3$, $-CON(R_3)_2$,

$R_3$, $R_4$ unabhängig voneinander Wasserstoff oder $(C_1-C_4)$-Alkyl,

$R_5$ Wasserstoff, Halogen, $(C_1-C_4)$-Alkylamino, Di-$(C_1-C_4)$-alkylamino, $(C_1-C_4)$-Alkyl, das gegebenenfalls ein oder mehrfach durch Halogen, $(C_1-C_3)$-Alkoxy oder durch $(C_1-C_3)$-Alkylthio substituiert ist, $(C_1-C_4)$-Alkoxy oder $(C_1-C_4)$-Alkylthio, Allyloxy- oder Propargyloxy,

$R_6$, $R_7$ unabhängig voneinander Wasserstoff, $(C_1-C_4)$-Alkyl, $(C_1-C_4)$-Halogenalkyl, Halogen oder $(C_1-C_2)$-Alkoxy-$(C_1-C_2)$-alkyl,

$R_8$ $(C_1-C_4)$-Alkyl, $(C_2-C_4)$-Alkenyl oder Benzyl,

$R_9$ $(C_1-C_4)$-Alkyl, das gegebenenfalls durch CN oder $(C_1-C_4)$-Alkoxycarbonyl substituiert ist, oder $(C_1-C_4)$-Alkoxy,

m die Zahl 1 bis 3,

n die Zahl 0 bis 2,

X eine Methingruppe oder Stickstoff

bedeuten, sowie deren Salze.

2. Verbindungen der Formel I von Anspruch 1, worin

$R_1$, $R_3$, $R_4$ Wasserstoff

$R_2$ ein Rest wie in Anspruch 1 außer $(C_1-C_4)$-Alkyl, $(C_1-C_4)$-Alkoxy oder $(C_1-C_4)$-Alkylthio

$R_5$ $(C_1-C_4)$-Alkyl, das gegebenenfalls einfach oder mehrfach durch Halogen, $(C_1-C_3)$-Alkoxy, $(C_1-C_3)$-Alkylthio substituiert ist, $(C_1-C_4)$-Alkoxy, Allyloxy oder Propargyloxy

$R_6$, $R_7$ Wasserstoff oder $(C_1-C_2)$-Alkyl

$R_8$ $(C_1-C_3)$-Alkyl und

m die Zahl 1 oder 2 bedeuten und sich der $R_2$-Rest in ortho-Stellung des Phenylringes befindet.

3. Verfahren zur Herstellung von Verbindungen der Formel I und deren Salze, dadurch gekennzeichnet, daß man

Verbindungen der Formel (II)

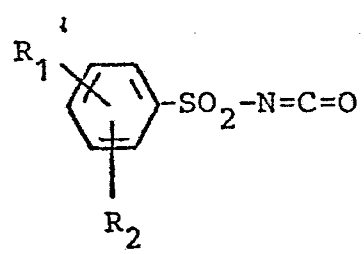

(II)

mit Verbindungen der Formel (III)

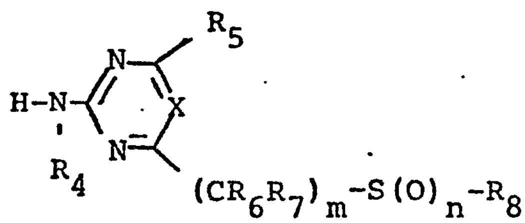

(III)

umsetzt, die erhaltenen Verbindungen der Formel I mit $R_3$ = H gegebenenfalls in $R_3$-Position alkyliert und gegebenenfalls im Falle n = O die erhaltenen Verbindungen der Formel I durch Umsetzung mit Oxidationsmitteln in die entsprechenden Sulfoxide der Formel I (n = 1) oder Sulfone der Formel I (n = 2) überführt und die erhaltenen Verbindungen der Formel I gegebenenfalls in ihre Salze überführt.

4. Herbizide und wachstumsregulierende Mittel, gekennzeichnet durch einen Gehalt an einer Verbindung der Formel I.

5. Verwendung von Verbindungen der Formel I zur Bekämpfung von unerwünschtem Pflanzenwuchs und zur Wachstumsregulierung.

6. Verfahren zur Bekämpfung von unerwünschtem Pflanzenwuchs bzw. zur Wachstumsregulierung, dadurch gekennzeichnet, daß man auf die zu behandelnden Pflanzen oder die Anbaufläche eine wirksame Menge einer Verbindung der Formel I aufbringt.

Patentansprüche: Österreich

1. Verfahren zur Herstellung von Verbindungen der Formel 1,

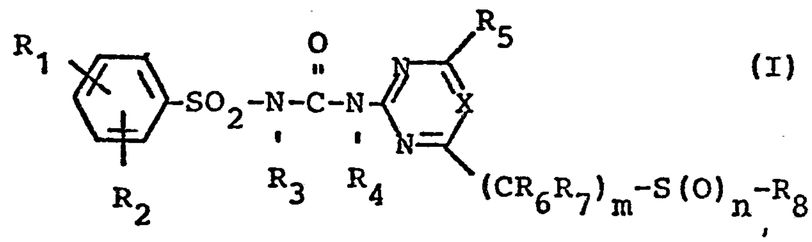

(I)

worin

$R_1$, $R_2$ unabhängig voneinander Wasserstoff, $(C_1-C_4)$-Alkyl, $(C_1-C_4)$-Alkoxy oder $(C_1-C_4)$-Alkylthio, die gegebenenfalls ein- oder mehrfach durch Halogen substituiert sind, Halogen, $NO_2$, $(C_1-C_4)$-Alkoxy-carbonyl, $(C_1-C_4)S(O)_n$-, $-SO_2N(R_9)(C_1-C_4$-Alkyl), $(C_1-C_4)$-Alkenyloxy, $(C_1-C_4)$-Alkinyloxy, $-OSO_2-(C_1-C_4)$-Alkyl, $-OSO_2CF_3$, $-CONHR_3$, $-CON(R_3)_2$,

$R_3$, $R_4$ unabhängig voneinander Wasserstoff oder $(C_1-C_4)$-Alkyl,

$R_5$ Wasserstoff, Halogen, $(C_1-C_4)$-Alkylamino, Di-$(C_1-C_4)$-alkylamino, $(C_1-C_4)$-Alkyl, das gegebenenfalls ein oder mehrfach durch Halogen, $(C_1-C_3)$-Alkoxy oder durch $(C_1-C_3)$-Alkylthio substituiert ist, $(C_1-C_4)$-Alkoxy oder $(C_1-C_4)$-Alkylthio, Allyloxy- oder Propargyloxy,

$R_6$, $R_7$ unabhängig voneinander Wasserstoff, $(C_1-C_4)$-Alkyl, $(C_1-C_4)$-Halogenalkyl, Halogen oder $(C_1-C_2)$-Alkoxy-$(C_1-C_2)$-alkyl,

$R_8$. $(C_1-C_4)$-Alkyl, $(C_2-C_4)$-Alkenyl oder Benzyl,

$R_9$ $(C_1-C_4)$-Alkyl, das gegebenenfalls durch CN oder $(C_1-C_4)$-Alkoxycarbonyl substituiert ist, oder $(C_1-C_4)$-Alkoxy,

m die Zahl 1 bis 3,

n die Zahl 0 bis 2,

X eine Methingruppe oder Stickstoff bedeuten,

sowie deren physiologisch verträglichen Salzen,

dadurch gekennzeichnet, daß man

Verbindungen der Formel (II)

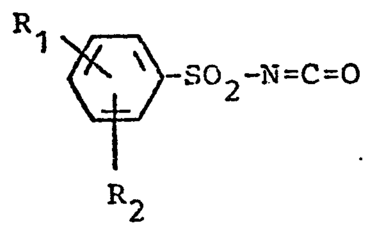

(II)

mit Verbindungen der Formel (III)

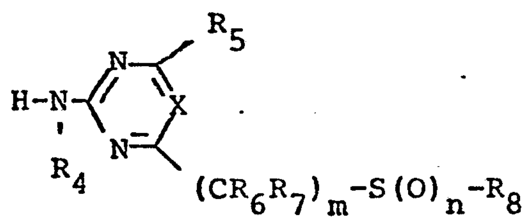

(III)

umsetzt, die erhaltenen Verbindungen der Formel I mit $R_3$ = H gegebenenfalls in $R_3$-Position alkyliert und gegebenenfalls im Falle n = 0 die erhaltenen Verbindungen der Formel I durch Umsetzung mit Oxidationsmitteln in die entsprechenden Sulfoxide der Formel I (n = 1) oder Sulfone der Formel I (n = 2) überführt und die erhaltenen Verbindungen der Formel I gegebenenfalls in ihre Salze überführt.

2. Verfahren gemäß Anspruch 1 zur Herstellung von Verbindungen der Formel I, worin

$R_1$, $R_3$, $R_4$ Wasserstoff

$R_2$ ein Rest wie in Anspruch 1 außer $(C_1-C_4)$-Alkyl, $(C_1-C_4)$-Alkoxy oder $(C_1-C_4)$-Alkylthio

$R_5$ $(C_1-C_4)$-Alkyl, das gegebenenfalls einfach oder mehrfach durch Halogen, $(C_1-C_3)$-Alkoxy, $(C_1-C_3)$-Alkylthio substituiert ist, $(C_1-C_4)$-Alkoxy, Allyloxy oder Propargyloxy

$R_6$, $R_7$ Wasserstoff oder $(C_1-C_2)$-Alkyl

$R_8$ $(C_1-C_3)$-Alkyl und

m die Zahl 1 oder 2 bedeuten und sich der $R_2$-Rest in ortho-Stellung des Phenylringes befindet.

3. Herbizide und wachstumsregulierende Mittel, gekennzeichnet durch einen Gehalt an einer Verbindung der Formel I.

4. Verwendung von Verbindungen der Formel I zur Bekämpfung von unerwünschtem Pflanzenwuchs und zur Wachstumsregulierung.

5. Verfahren zur Bekämpfung von unerwünschtem Pflanzenwuchs und zur Wachstumsregulierung, dadurch gekennzeichnet, daß man auf die zu behandelnden Pflanzen oder die Anbaufläche eine wirksame Menge einer Verbindung der Formel I aufbringt.

Europäisches Patentamt

**EUROPÄISCHER RECHERCHENBERICHT**

0134477
Nummer der Anmeldung

EP 84107831.4

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
| A | EP - A2 - 0 001 514 (DU PONT)<br>* Zusammenfassung *<br>-- | 1,3,4 | C 07 D 251/16<br>C 07 D 239/42<br>A 01 N 47/36 |
| A | EP - A2 - 0 001 515 (DU PONT)<br>* Zusammenfassung *<br>-- | 1,3,4 | |
| A | EP - A1 - 0 004 163 (DU PONT)<br>* Zusammenfassung *<br>-- | 1,3,4 | |
| A | EP - A1 - 0 010 560 (DU PONT)<br>* Zusammenfassung *<br>-- | 1,3,4 | |
| A | EP - A1 - 0 048 143 (DU PONT)<br>* Zusammenfassung *<br>-- | 1,3,4 | RECHERCHIERTE SACHGEBIETE (Int. Cl.4) |
| A | US - A - 4 301 286 (SCHWING et al.)<br>* Anspruch 1 *<br>---- | 1,4 | C 07 D 251/00<br>C 07 D 239/00 |

Der vorliegende Recherchenbericht wurde für alle Patentansprüche erstellt.

| Recherchenort | Abschlußdatum der Recherche | Prüfer |
|---|---|---|
| WIEN | 10-09-1984 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82